# EUROPEAN PATENT SPECIFICATION

(11) **EP 3 217 874 B1**
(45) Date of publication and mention of the grant of the patent: **09.03.2022**
(21) Application number: 15791596.8
(22) Date of filing: 07.11.2015
(51) Int. Cl.: A61B 5/08, A61B 5/11, A61B 5/113

(54) **APPARATUS AND METHOD FOR ASSESSING THE SEVERITY OF CHRONIC OBSTRUCTIVE PULMONARY DISEASE, COPD, IN A SUBJECT**
VORRICHTUNG UND VERFAHREN ZUR BESTIMMUNG DES SCHWEREGRADES VON CHRONISCH OBSTRUKTIVER LUNGENERKRANKUNG BEI EINER PERSON
APPAREIL ET PROCÉDÉ PERMETTANT D'ÉVALUER LA GRAVITÉ D'UNE MALADIE PULMONAIRE OBSTRUCTIVE CHRONIQUE, LA BPCO, CHEZ UN SUJET

(30) Priority: 12.11.2014 EP 14192818
(43) Date of publication of application: 20.09.2017
(73) Proprietor: Koninklijke Philips N.V., 5656 AG Eindhoven (NL)
(72) Inventor: DELLIMORE, Kiran Hamilton J., NL-5656 AE Eindhoven (NL); KUENEN, Maarten, NL-5656 AE Eindhoven (NL); DE SAMBER, Marc Andre, NL-5656 AE Eindhoven (NL); AARTS, Ronaldus Maria, NL-5656 AE Eindhoven (NL)
(74) Representative: Philips Intellectual Property & Standards
(86) International application number: PCT/EP2015/075999
(87) International publication number: WO 2016/075054

(56) References cited:
- WO-A1-2010/132850
- WO-A1-2014/130944
- US-A1- 2010 275 921

## Description

### TECHNICAL FIELD OF THE INVENTION

The invention relates to an apparatus and computer program product for assessing the severity of chronic obstructive pulmonary disease, COPD, in a subject.

### BACKGROUND TO THE INVENTION

Chronic obstructive pulmonary disease (COPD) is a progressive lung disease characterized by persistent airflow limitation, which is associated with an enhanced chronic inflammatory response in the airways and in the lung due to noxious particles or gases (for example inhaled cigarette smoke or other noxious gases such as smoke from biomass fuels). COPD is a leading cause of morbidity and mortality worldwide, which results in a very significant economic and social burden that is increasing each year. COPD was responsible for more than 2.5 million deaths worldwide in 2000 alone. COPD currently ranks as the third leading cause of death in the United States and is projected to have the fifth leading burden of disease worldwide by the year 2020. Since COPD often develops in long-time smokers, typically in middle age, it is often associated with a variety of comorbid diseases related to smoking or aging. Among the most common comorbidities are cardiovascular disease (including ischemic heart disease, heart failure, atrial fibrillation and hypertension), lung cancer, metabolic syndrome and diabetes, osteoporosis and respiratory infections (which are usually very severe). COPD is (currently) an incurable disease, which can be managed or stabilized by medication (i.e. drug therapy), surgery, and through lifestyle changes (e.g. smoking cessation and exercise). Bronchodilators (usually β2 agonists and anticholinergics) are the main medications used to treat COPD. They can be both fast acting (4-6 hours; e.g., ipratropium, salbutamol and terbutaline) or long-acting (over 12 hours; e.g., tiotropium, salmeterol and formoterol). For the treatment and prevention of acute exacerbation events, corticosteroids are also often administered. Long-term antibiotics, especially those from the macrolide class (e.g., erythromycin) may also be prescribed to COPD patients to reduce the frequency of exacerbations.

An exacerbation of COPD is defined as an acute event which is characterized by a worsening of the patient's respiratory symptoms that is beyond the typical day to day variations and which leads to a change in the therapeutic drug medication. The risk of exacerbation increases as airflow limitation worsens, and is best predicted from a history of previous exacerbation events.

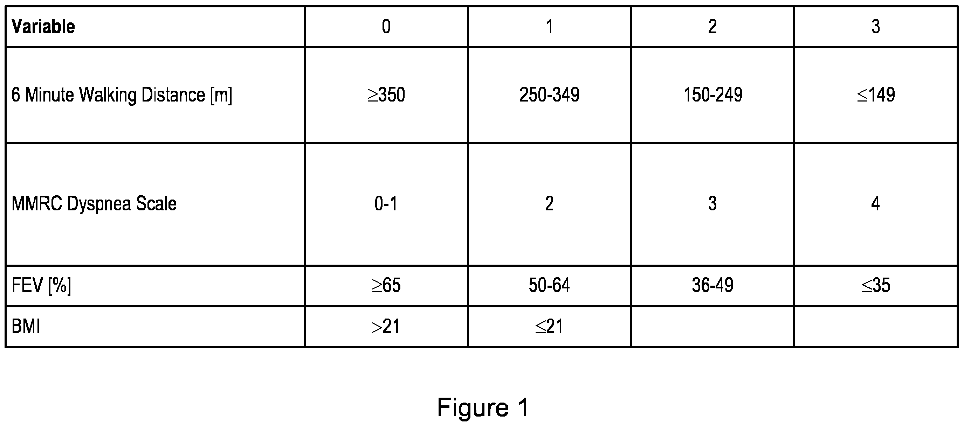

An increased risk of mortality in patients with COPD is linked to several clinically assessable variables including force exhaled volume, FEV (the amount of air that can be forcibly exhaled in a certain time period after a full inhalation), exercise tolerance, peak oxygen consumption, weight loss and reduction in arterial oxygen. Typically COPD disease severity is assessed by using a combination of these variables in a composite index called the BODE (Body mass index, Obstruction, Dyspnea and Exercise) score. The BODE score is considered to be a better predictor for patient survival than any of the individual variables composing the score. The BODE index is derived as shown in Figure 1. It is based on the Body Mass Index (>21 = 0 points and ≤21 = 1 point) and the following variables which are scored on a scale of 0 to 3 points: FEV after taking a bronchodilator (≥65 % = 0 points, 50-64% = 1 point, 36-49% = 2 points and ≤35% = 3 points), 6 minute Walking Distance (≥350 m = 0 points, 250-349 m = 1 point, 150-249m = 2 points and ≤149m = 3 points) and Modified Medical Research Council Scale (MMRC) Dyspnea Scale (Dyspneic on strenuous exercise or on walking up a slight hill = 0 points, Dyspneic on walking on level ground; must stop occasionally due to breathlessness = 1 points, Must stop due to breathlessness after walking 100 yards or after a few minutes = 2 points, and Cannot leave house; breathless on dressing/undressing = 3 points). BODE scores of 0 (less impaired) to 10 (most impaired) are stratified into 4 quartiles, which discriminate mortality risk.

Despite the use of BODE scoring, the clinical assessment and therapeutic management of COPD patients remains extremely difficult for various reasons. The many challenges often encountered include, for example, poor drug compliance, promoting lifestyle changes (e.g. smoking cessation, increasing exercise level and encouraging psychological and social well-being) as well as patient discomfort during clinical tests for COPD (e.g., the FEV test used for BODE scoring is extremely unpleasant for the patient). Moreover, during clinical assessment of COPD severity it is necessary for a highly trained clinician to be present to perform BODE scoring which adds to the cost of disease management and results in less frequent assessments. In addition, mild COPD is difficult to detect and frequently goes undiagnosed.

Therefore, there is a significant clinical need for a method to detect COPD at an early stage, and, once COPD is diagnosed, to provide continuous or on-demand, objective, patient-friendly and reliable methods for the assessment of COPD severity to improve management of the disease.

US 2010/0275921 A1 discloses devices and systems that provide methods of detecting a severity change in respiratory insufficiency (RI) or COPD condition of a patient. In an example embodiment, a detection monitoring device determines one or more severity change indicators based on a measure of supplied pressure or other representative measure determined by the device. The supplied pressure may optionally be determined during pressure treatment that satisfies a target ventilation. The supplied pressure or representative data may be compared to one or more thresholds that are selected to represent a change in the condition of the RI or COPD patient such as an exacerbation of a prior condition. Results of the comparisons may trigger one or more warnings or messages to notify a patient or physician of a pending change to the patient's RI or COPD condition so that the patient may more immediately seek medical attention to treat the condition.

WO 2014/130944 A1 discloses a method and system for managing a chronic medical condition, such as COPD. The method includes generating a baseline score for a patient; recording an exacerbation severity for a plurality of symptoms; weighting the recorded exacerbation severities; determining an exacerbation score relative to the baseline score based on at least one of the recorded or weighted exacerbation severities; assigning the exacerbation score to a category; publishing the exacerbation score and assigned category for review by a physician or medical professional; and transmitting a treatment plan to the patient, the treatment plan being prescribed by a physician and based at least in part on the exacerbation score and/or assigned category.

### SUMMARY OF THE INVENTION

As noted above, the clinical assessment and therapeutic management of patients (also referred to herein as subjects or users) with COPD is very challenging for clinicians and care givers. The available tools not particularly practical or user friendly, and cannot easily provide continuous or on-demand objective assessment of COPD disease severity and its evolution.

Therefore, according to one aspect of the invention, there is provided a computer program product of assessing the severity of chronic obstructive pulmonary disease, COPD, in a subject, the computer program product comprising a computer readable medium having computer readable code embodied therein, the computer readable code being configured such that, on execution by a suitable computer or processor, the computer or processor is caused to perform determining by means of an accelerometer measurements of a breathing rate of the subject, an activity level of the subject, a measure of the respiratory effort of the subject and a measure of the severity or intensity of coughing by the subject; and combining the measurements of the breathing rate, the activity level, the measure of the respiratory effort and the measure of the severity or intensity of coughing to determine a score representing the severity of COPD in the subject.

In preferred embodiments the breathing rate of the subject, the activity level of the subject, the measure of the respiratory effort of the subject and the measure of the severity or intensity of coughing by the subject are determined from measurements from a the accelerometer only.

In alternative embodiments, the breathing rate of the subject, the activity level of the subject, the measure of the respiratory effort of the subject and the measure of the severity or intensity of coughing by the subject are determined from measurements from the accelerometer and at least one additional sensor. In some embodiments the at least one additional sensor is a sensor for measuring the motion and/or position of the subject.

In some embodiments the measure of the respiratory effort is a ratio of inhaled to exhaled breath of the subject. In some embodiments the measure of the severity or intensity of coughing is a measure of the number of coughs over a predetermined period.

In some embodiments the step of combining the measurements to determine a score representing the severity of COPD in the subject comprises comparing each of the measurements of the breathing rate, the activity level, the measure of the respiratory effort and the measure of the severity or intensity of coughing to a respective severity range to determine a score for each of the measurements; and adding the determined scores to determine the score representing the severity of COPD in the subject.

In alternative embodiments the step of combining the measurements to determine a score representing the severity of COPD in the subject comprises analyzing the measurements of the breathing rate, the activity level, the measure of the respiratory effort and the measure of the severity or intensity of coughing using a classification algorithm to determine the score.

In some embodiments the computer program product further comprises the step of calibrating the score representing the severity of COPD in the subject against a Body mass index, Obstruction, Dyspnea and Exercise, BODE, score.

In some embodiments the step of calibrating the score representing the severity of COPD against a BODE score comprises determining a BODE-equivalent score from the measurements of the breathing rate, the activity level, the measure of the respiratory effort and the measure of the severity or intensity of coughing, and measurements of the forced exhaled volume, FEV, and body mass index, BMI, of the subject; and comparing the determined BODE-equivalent score to a BODE score obtained from measurements of the FEV, BMI, the distance walked by the subject in six minutes and a measure of the dyspnea of the subject on the Modified Medical Research Council Scale, MMRC, Dyspnea Scale.

In some embodiments the computer program product further comprises the step of normalizing each of the measurements of the breathing rate, the activity level, the measure of the respiratory effort and the measure of the severity or intensity of coughing using initial measurements of the breathing rate, the activity level, the measure of the respiratory effort and the measure of the severity or intensity of coughing.

In some embodiments the step of combining the measurements to determine a score representing the severity of COPD in the subject uses an indication of the ambulatory state of the subject.

In some embodiments the computer program product further comprises the step of comparing the measure of the intensity or severity of coughing to a threshold value to determine if there is a high risk of an exacerbation event or if an exacerbation event is ongoing.

In some embodiments the computer program product further comprises the step of detecting when the subject coughs or gasps; and temporarily interrupting the step of determining measurements or the use of the measurements in the step of combining when the subject coughs or gasps.

According to another aspect, there is provided an apparatus for assessing the severity of chronic obstructive pulmonary disease, COPD, in a subject, the apparatus comprising a control unit configured to determine by means of an accelerometer measurements of a breathing rate of the subject, an activity level of the subject, a measure of the respiratory effort and a measure of the intensity or severity of coughing, and to combine the measurements of the breathing rate, the activity level, the measure of respiratory effort and the measure of the intensity or severity of coughing to determine a score representing the severity of COPD in the subject.

In preferred embodiments the control unit is configured to determine the measurements of the breathing rate of the subject, the activity level of the subject, the measure of the respiratory effort of the subject and the measure of the severity or intensity of coughing by the subject from measurements from the accelerometer only.

In alternative embodiments the apparatus further comprises the accelerometer and at least one additional sensor, and the control unit is configured to determine the measurements of the breathing rate of the subject, the activity level of the subject, the measure of the respiratory effort of the subject and the measure of the severity or intensity of coughing by the subject from measurements from the accelerometer and the at least one additional sensor. In some embodiments the at least one additional sensor is a sensor for measuring the motion and/or position of the subject.

In some embodiments the measure of the respiratory effort is a ratio of inhaled to exhaled breath of the subject. In some embodiments the measure of the severity or intensity of coughing is a measure of the number of coughs over a predetermined period.

In some embodiments the control unit is configured to combine the measurements to determine a score representing the severity of COPD in the subject by comparing each of the measurements of the breathing rate, the activity level, the measure of the respiratory effort and the measure of the severity or intensity of coughing to a respective severity range to determine a score for each of the measurements; and adding the determined scores to determine the score representing the severity of COPD in the subject.

In alternative embodiments the control unit is configured to combine the measurements to determine a score representing the severity of COPD in the subject by analyzing the measurements of the breathing rate, the activity level, the measure of the respiratory effort and the measure of the severity or intensity of coughing using a classification algorithm to determine the score.

In some embodiments the control unit is further configured to calibrate the score representing the severity of COPD in the subject against a Body mass index, Obstruction, Dyspnea and Exercise, BODE, score.

In some embodiments the control unit is configured to calibrate the score representing the severity of COPD against a BODE score by determining a BODE-equivalent score from the measurements of the breathing rate, the activity level, the measure of the respiratory effort and the measure of the severity or intensity of coughing, and measurements of the forced exhaled volume, FEV, and body mass index, BMI, of the subject; and comparing the determined BODE-equivalent score to a BODE score obtained from measurements of the FEV, BMI, the distance walked by the subject in six minutes and a measure of the dyspnea of the subject on the Modified Medical Research Council Scale, MMRC, Dyspnea Scale.

In some embodiments the control unit is further configured to normalize each of the measurements of the breathing rate, the activity level, the measure of the respiratory effort and the measure of the severity or intensity of coughing using initial measurements of the breathing rate, the activity level, the measure of the respiratory effort and the measure of the severity or intensity of coughing.

In some embodiments the control unit is configured to combine the measurements to determine a score representing the severity of COPD in the subject using an indication of the ambulatory state of the subject.

In some embodiments the control unit is further configured to compare the measure of the intensity or severity of coughing to a threshold value to determine if there is a high risk of an exacerbation event or if an exacerbation event is ongoing.

In some embodiments the control unit is further configured to detect when the subject coughs or gasps; and temporarily interrupt the determining of measurements or the combination of the measurements to determine the score representing the severity of the COPD in the subject when the subject coughs or gasps.

According to yet another aspect, there is provided a system for assessing the severity of chronic obstructive pulmonary disease, COPD, in a subject, the system comprising a sensor for measuring characteristics of the subject; and an apparatus as described above, wherein the control unit determines the measurements from the output of the sensor.

Thus, one problem addressed by the invention described above is the fact that the conventional BODE score is a point measurement and requires many different sensors and hardware which is cumbersome and inconvenient for the subject to use. Therefore it is not suited to continuous monitoring of COPD severity, which means that deterioration of the disease (i.e., decrease in respiratory function) is not easily detected. BODE testing is further inconvenient for the subject since they must travel to the hospital or clinic to be evaluated, which disrupts their daily life, and it requires a highly-skilled clinician to be present to perform the diagnosis. Also, testing in a stressful situation, after travel to hospital and in a hospital environment, might negatively affect the test results.

Embodiments of the invention allow for earlier detection of mild COPD and better management of COPD drug therapy since the apparatus and computer program product are unobtrusive, they can provide continuous or on-demand assessments and utilize a single, easy-to-use apparatus. It can also be used to establish an historical overview of patient-specific effectiveness of bronchodilators in reducing breathing problems and thereby enable patient-specific dosing. In addition, with 24 hour (i.e., continuous) monitoring, embodiments provide that it is possible to measure drug timing, monitor compliance, and estimate the optimal times to use a bronchodilator or corticosteroid to prevent the occurrence or minimize the severity of an exacerbation.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a table illustrating the derivation of the BODE index;
Figure 2 is a block diagram of an apparatus according to an aspect of the invention;
Figure 3 is a flow chart illustrating computer program product steps of assessing the severity of COPD in a subject;
Figure 4 is a table illustrating the derivation of a COPD severity index according to an embodiment; and
Figure 5 is a series of graphs and a flow chart illustrating how breathing rate can be determined from accelerometer measurements according to an embodiment;
Figure 6 is a set of graphs illustrating how the ratio of inhaled to exhaled breath can be determined from accelerometer measurements according to an embodiment;
Figure 7 is a graph illustrating how the activity level can be determined from accelerometer measurements according to an embodiment;
Figure 8 is a graph illustrating how the number of coughs in a predetermined time period can be determined from accelerometer measurements in an embodiment; and
Figure 9 is a diagram that provides an overview of the sensors and physiological parameters that can be used for determining COPD severity in according with various embodiments of the invention.

### DETAILED DESCRIPTION OF THE PREFERRED EMBODIMENTS

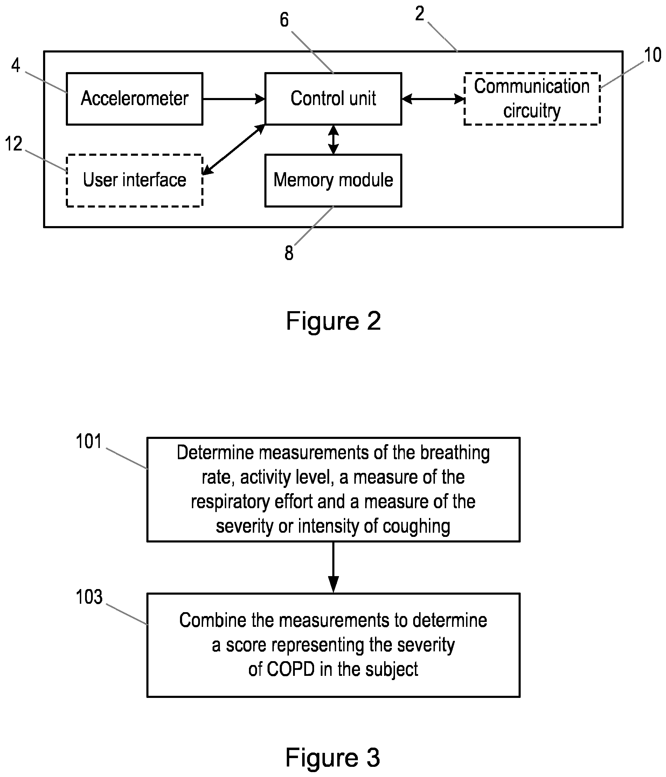

Figure 2 is a block diagram illustrating an apparatus 2 according to an aspect of the invention. The apparatus 2 is to be worn or carried by a subject and comprises a sensor for measuring the movements or motion of the subject. In particular, the sensor is for measuring at least the motion or movements of the chest or other part of the body of the subject so that characteristics of the breathing of the subject (e.g. breathing rate, a measure of respiratory effort such as a ratio of inhalation to exhalation, and a measure of the severity or intensity of coughing, such as the number of coughs in a predetermined time period) can be determined from the measurements.

Preferably, the sensor is an accelerometer 4, although those skilled in the art will appreciate that other types of sensors can be used to obtain the measurements required for assessing COPD severity according to the invention. In some embodiments the accelerometer 4 is a three-dimensional accelerometer that measures the accelerations in three dimensions, but in other embodiments the accelerometer 4 comprises three one-dimensional accelerometers arranged orthogonally to each other. The accelerometer 4 measures the magnitude and direction of the acceleration acting on the apparatus 2 and outputs an acceleration signal indicating the acceleration in three dimensions to a control unit 6. The accelerometer 4 can operate according to any desired operating or sampling frequency to measure the acceleration, for example 50 Hz.

In preferred embodiments, the only sensor required to obtain the measurements for assessing the COPD severity according to the invention is the accelerometer 4 (i.e. the output from a single or the same sensor 4 is used to determine the parameters according to the invention). These embodiments provide a low-cost, simple, apparatus 2 for assessing COPD severity. However, in other embodiments, the apparatus 2 can comprise one or more additional sensors for obtaining measurements that can be used to determine the breathing characteristics and other characteristics of the subject used in the assessment of COPD severity, and/or that can be used to determine other parameters for the subject that may or may not be used to assess the COPD severity. Such sensors can include sensors for measuring the motion and/or position of the subject, for example a gyroscope, a magnetometer, a satellite positioning system (e.g. GPS) receiver (any some or all of which can be used as part of assessing the activity or activity level of the subject), a microphone for measuring the sound of the subject's breathing (and which can be used to determine the breathing rate and/or determine when the subject coughs) and/or a temperature sensor (for measuring the subject's temperature).

The control unit 6 controls the operation of the apparatus 2 according to the invention. The control unit 6 can comprise one or more processors, processing units, multicore processors or processing modules. The apparatus 2 further comprises a memory module 8 for storing computer readable program code that can be executed by the control unit 6 to perform the steps caused to be performed by the computer program product according to the invention. The memory module 8 can also be used to store the sensor (acceleration) measurements before, during and after processing by the control unit 6 and any intermediate products of the processing.

In this illustrated embodiment of the invention, the apparatus 2 comprises a single unit or device that is worn or carried by the subject and that collects and processes the acceleration measurements (in the control unit 6) to determine the COPD severity. In alternative embodiments, the processing of the measurements can be performed in a control unit that is remote from the accelerometer 4 (for example in a unit that is worn on a different part of the body of the subject, in a base unit or computer that can be located in the subject's home, or a remote server located in the premises of a healthcare service provider), in which case the apparatus 2 will comprise a sensor unit to be worn by the subject (that is similar to that shown in Figure 2) and that comprises suitable transmitter, transceiver or communication circuitry 10 for transmitting the measurements to a control unit in the remote unit. In either embodiment, the apparatus 2 can be part of a COPD severity monitoring system which comprises a display or other visual indicator (that can themselves be part of or separate from the apparatus 2) that can be used to indicate the determined COPD severity score to the subject or a clinician.

In preferred embodiments of the invention the apparatus 2 is sized and/or shaped so that it can be worn or carried on the upper body of the subject, for example on the chest, thorax or abdomen (and in particular in the subclavian chest area or on the abdomen below the diaphragm). The apparatus 2 can be provided with some means to enable the apparatus 2 to be held in contact with the subject so that the sensor 4 can obtain the required measurements of the motion of the subject to enable the breathing characteristics to be determined. For example, the apparatus 2 can be provided with a belt or strap, or the apparatus 2 can be part of an adhesive patch.

In practical implementations, the apparatus 2 may comprise other or further components to those shown in Figure 2 and described above, such as a user interface 12 that allows the subject to activate and/or operate the apparatus 2, and a power supply, such as a battery, for powering the apparatus 2. The user interface 12 may comprise one or more components that allow a user (e.g. the subject) to interact and control the apparatus 2. As an example, the one or more user interface components could comprise a switch, a button or other control means for activating and deactivating the apparatus 2 and/or measurement process. The user interface components can also or alternatively comprise a display, or other visual indicator (such as a light) for providing information to the subject about the operation of the apparatus 2, including displaying the determined COPD severity score. Likewise, the user interface components can comprise an audio source for providing audible feedback to the subject about the operation of the apparatus 2, including an audible indication of the determined COPD severity score.

It will be appreciated that in some embodiments the apparatus 2 is a dedicated apparatus for determining COPD severity (i.e. the sole purpose of the apparatus 2 is to determine the COPD severity). However, in other embodiments, the COPD severity score according to the invention can be determined by any type of apparatus or device that comprises a sensor 4 that is able to obtain the required measurements for determining the COPD severity score. For example, the apparatus 2 can be a user-worn or carried activity or motion monitor that monitors the physical activity of the subject, for example for personal fitness purposes, for supporting injury or fall prevention, or for detecting falls. In some embodiments, the apparatus 2 can be in the form of a smart phone executing a suitable application.

To assess the severity of COPD, the invention makes use of various clinical indicators of COPD severity that can be easily measured by a simple apparatus 2, and that can be measured simultaneously and continuously, if required.

Four parameters of a subject have been identified that can be combined and used to assess COPD severity. These parameters are the activity level of the subject, the breathing rate of the subject, a measure of respiratory effort (such as the ratio of inhaled to exhaled breath) and a measure of the severity or intensity of coughing (such as the number of coughs in a predetermined period of time, for example a minute or hour). These parameters are all clinically known to be highly sensitive for the determination of COPD severity and are closely linked to respiratory system function. The close correlation between the parameters is illustrated for instance by the fact that a subject with moderate to severe COPD will almost always experience an increased breathing rate during physical activity along with an increased coughing rate and decreased inhaled to exhaled breath ratio.

Although these parameters are sensitive to COPD severity, individually they do not provide a reliable measure of COPD sensitivity in a subject. Therefore, the invention provides that measurements of these four parameters are combined to determine a COPD severity score, which is similar in concept to the BODE score described above.

Since these parameters can be measured continuously or frequently throughout the day, the invention provides significant advantages over existing solutions as it can be used to monitor the progression of COPD severity over time. Moreover, continuous monitoring allows daily variations and artefacts to be distinguished better than a point-of-care solution and it permits long-term COPD disease progression to be assessed. With continuous or frequent monitoring the COPD severity score according to the invention can provide early warnings of impending exacerbations and be used for COPD therapy management, e.g. medication dosing and exercise scheduling. In some embodiments, to improve and maintain the clinical reliability of the invention, a BODE-equivalent score can be obtained from the four parameters set out above and compared to a standard BODE score obtained during a scheduled out-patient, point-of-care visit with a doctor.

The flow chart in Figure 3 illustrates steps performed by a computer program product of assessing the severity of COPD in a subject according to the invention. In a first step, step 101, measurements of the breathing rate, the activity level, a measure of respiratory effort and a measure of the severity or intensity of coughing are determined. As noted above, these measurements are preferably determined from the output of a single sensor 4, such as an accelerometer.

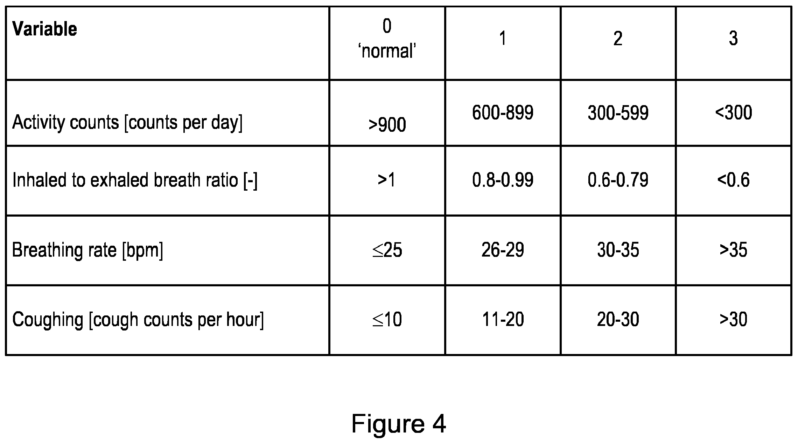

Then, in step 103, the measurements of the parameters are combined to determine a score representing the severity of the COPD in the subject. Those skilled in the art will appreciate that there are a number of ways in which a COPD severity score can be formed from the measurements. In some embodiments, severity ranges can be defined for measurements of each of the four parameters, with score values being assigned to each severity range, and the COPD severity score obtained by summing the score values associated with the measurements of each parameter. This way of determining the COPD severity score is illustrated by the exemplary table in Figure 4 (in which the measure of respiratory effort is represented by a ratio of inhalation to exhalation and the measure of the severity or intensity of coughing is represented by the number of coughs over an hour) and is similar to the way in which the BODE score is determined.

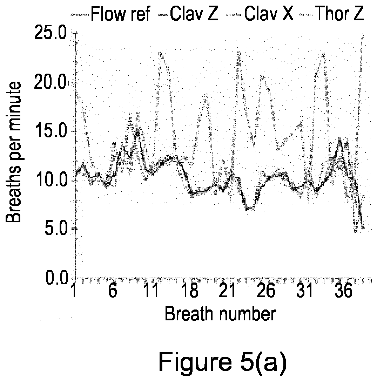

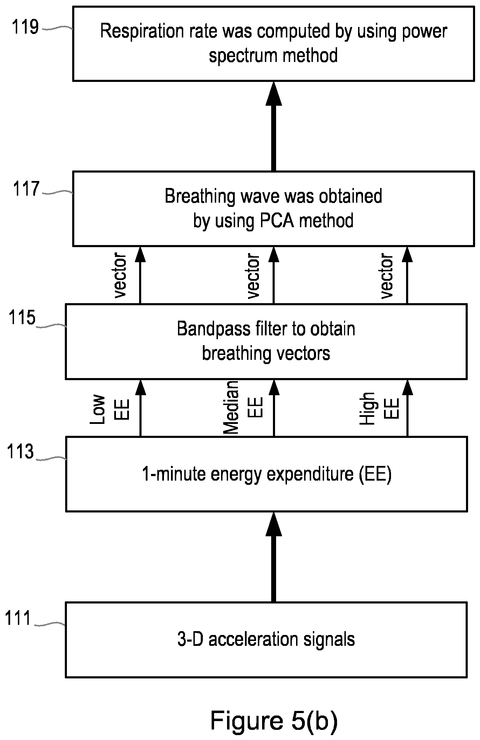

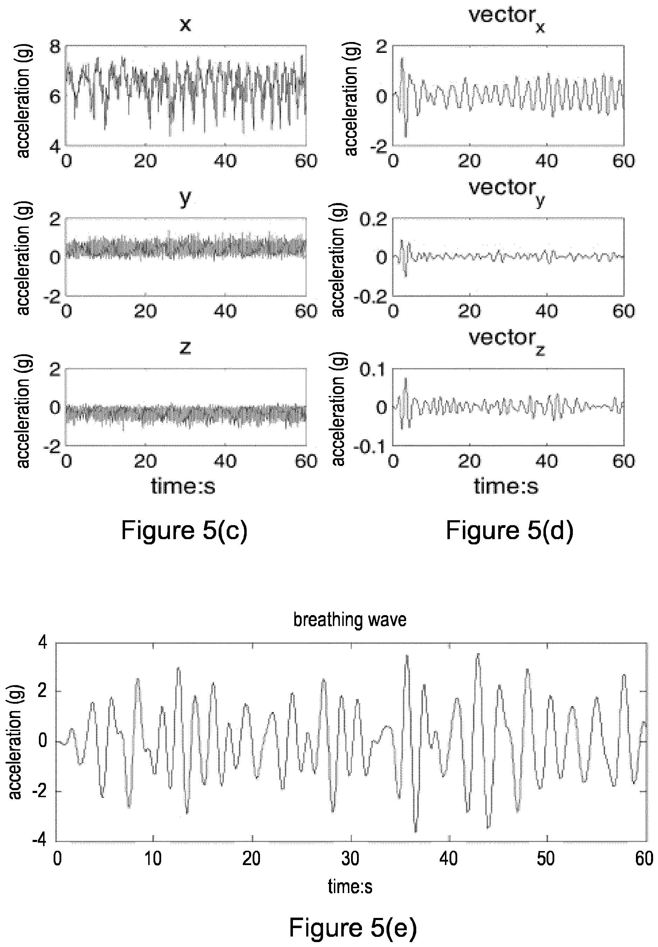

Figures 5-8 illustrate exemplary ways in which the breathing rate, a measure of respiratory effort (in particular the ratio of inhaled to exhaled breath), activity level and a measure of the severity or intensity of coughing (in particular the number of coughs in a predetermined time period) can be determined from accelerometer measurements.

The breathing rate is typically measured in terms of the number of breaths per minute (bpm). In some embodiments, the breathing rate can be determined from the acceleration measurements by applying a filter to the raw accelerometer signal in the frequency domain that passes frequencies in a range corresponding to typical or possible breathing rate. This is shown in Figure 5(a). For example, the filter can pass frequencies in the range of 0.15-0.70 Hz (corresponding to breathing rates between 9-42 bpm). It will be appreciated that a higher breathing rate is associated with more severe levels of COPD and decreased respiratory function.

Figure 5(b) illustrates an exemplary way of processing the acceleration signals to determine the breathing rate. The signals from the accelerometer 4 (111 and shown in Figure 5(c)) are processed (in 113) to determine the energy expenditure over a predetermined time period (1 minute in this example). Those skilled in the art will appreciate that 113 can be performed in a number of ways. For example the type of activity that the subject is performing (e.g. walking, riding a bike, etc.) can be detected and a given energy expenditure for the particular activity assumed. Alternatively, the physical activity energy expenditure can be estimated directly from the accelerometer signals. The result of 113 is divided into three parts, representing low energy expenditure, medium energy expenditure and high energy expenditure, and each is bandpass filtered to obtain breathing vectors (115). The bandpass filter should pass at least frequencies corresponding to typical breathing rates (e.g. 12-15 breaths per minute, bpm, for normal adults), and perhaps some of the harmonics of those frequencies as well. The breathing vectors are shown in Figure 5(d). In 117 the vectors are processed using a principle component analysis (PCA) method to obtain a 'breathing wave' (as shown in Figure 5(e)). Then, in 119, the respiration rate is computed by analyzing the breathing wave using a power spectrum analysis technique.

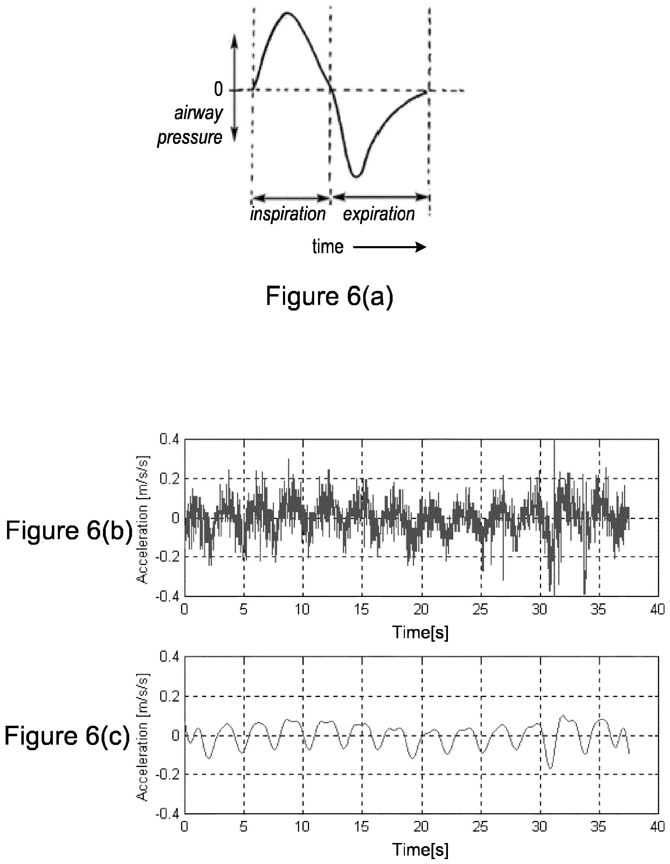

The ratio of inhaled to exhaled breath is the ratio of the time taken to inhale to the time taken to exhale. Like the breathing rate, in some embodiments the ratio of inhaled to exhaled breath can be determined from the acceleration measurements by applying a filter to the raw accelerometer signal to distinguish the time period required for inspiration versus expiration. Figure 6(a) shows measurements of airway pressure as a subject inhales and exhales (it will be appreciated that this graph is provided to illustrate how the ratio is calculated, in the preferred embodiments the ratio of inhaled to exhaled breath is measured from accelerometer measurements as described below, although in less preferred embodiments an air pressure sensor could be used instead). During inhalation, the chest will generally move outwards; during exhalation, the chest will move in the opposite direction. Changes in the movement direction can be identified in the accelerometer measurements as zero crossings (i.e. where the acceleration in the direction parallel to the movement of the chest is zero). Therefore the period between zero crossings represents the duration of both inhalation and exhalation. Figure 6(b) shows an exemplary signal from an axis of the accelerometer that is optimally attached to the subject so that the inward and outward movements of the chest or abdomen are represented in the signal. The signal is low pass filtered to obtain the signal in Figure 6(c), from which the zero crossings can easily be identified. The cut-off frequency for the filter can be selected so that it passes frequencies corresponding to typical breathing rates (e.g. a typical breathing rate for a normal adult is 12-15 bpm, so the cut-off frequency should pass these frequencies). The periods between the zero crossings represent the duration of the inhalation or exhalation.

For patients with COPD greater effort is required for exhalation than inhalation, which means that there is a significant difference in the momentum change (i.e., acceleration) of the chest and abdomen during inhalation and exhalation, which can be easily detected by the accelerometer. Inhaled to exhaled breath ratios less than 1 represent higher COPD severity than ratios greater than 1. It will be appreciated that measures of the respiratory effort other than the ratio of inhalation to exhalation can be determined in alternative embodiments of the invention. For example, measures representing the energy, intensity or strength of the respiration can be used, such as the amplitude, the root-mean-squared, RMS, value, the average or the Teager energy ratio of a respiration signal isolated or separated from the acceleration signal.

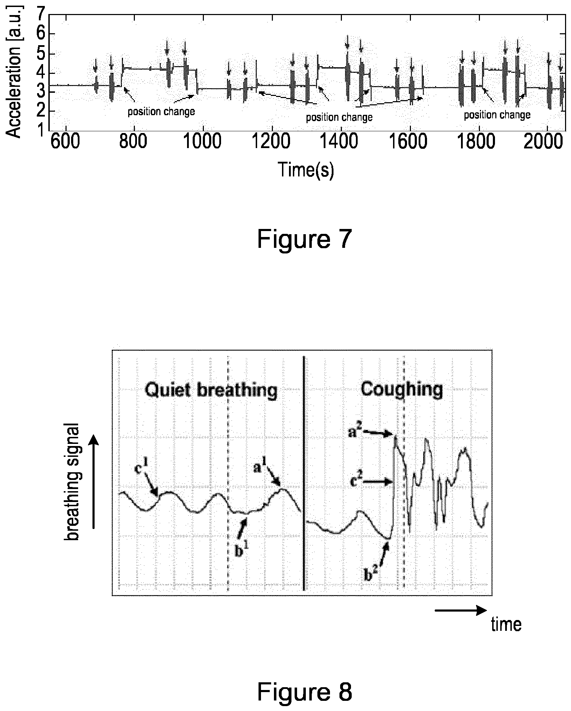

The activity level of the subject represents the physical activity/movement level of the subject in a given period of time. Those skilled in the art will be aware of various ways in which the activity level of a subject can be determined. In some embodiments, the activity level can be detected by quantifying the amplitude and frequency of the detected accelerations in time intervals of, for example, 1 or 2 minutes, to determine whether the subject is moving or exerting physical effort. An exemplary acceleration signal that has been processed to detect activities is shown in Figure 7. The y-axis represents acceleration, and the arrows indicate detected activities. The activity level can be used to determine the state of the subject at the time that their breathing rate and the ratio of inhaled versus exhaled breathing effort is measured, and also to give an indication of how active or sedentary a subject is during the course of the day. The paper "The technology of accelerometry-based activity monitors" by Chen et al. Med Sci Sports Exercise 2005 Nov; 37(11 Suppl): S490-500 describes a suitable approach for calculating the activity level of the subject.

The number of coughs over a predetermined period of time (e.g. per minute, per hour, etc.), which can also be referred to as a 'cough rate', can be determined by detecting artefacts in the acceleration measurements. In particular artefacts can be detected in the filtered accelerometer measurements used to detect the breathing rate above (e.g. as shown in Figure 5(e)) or the filtered acceleration measurements used to determine the measure of respiratory effort (e.g. as shown in Figure 6(c)). An extract from a suitable signal is shown in Figure 8. In Figure 8, the parameter a represents the maximum amplitude of the breathing signal, the parameter b represents the minimum amplitude and the parameter c represents the average amplitude. Coughing can be detected using a classification algorithm that detects short, high impulse, large-amplitude motion signals as coughs, and longer, low-amplitude motion signals as gasps. It will be appreciated that measures of the intensity or severity of coughing other than the number of coughs over a predetermined period of time can be determined in alternative embodiments of the invention. For example, measures representing the energy, intensity or strength of the coughing can be used, such as the amplitude, the root-mean-squared, RMS, value, the average or the Teager energy ratio of a signal representing the coughing that is isolated or separated from the acceleration signal.

The number of coughs over a certain period of time, for example 1 hour, can be used to assess the respiratory condition and can also be used as an aid in the prediction of exacerbation events. In particular, a high cough count (high compared to an average for the COPD population or high for the specific subject being assessed) can be an indicator that the subject may be at risk of, or experiencing, an exacerbation in their symptoms. Therefore, the cough count or other measure of the intensity or severity of coughing can be compared to a threshold value to determine if there is a high risk of an exacerbation event or an exacerbation event is ongoing.

In some embodiments, when a cough or gasp is detected, the control unit 6 may temporarily interrupt the calculation or use of the other parameters in determining a COPD severity score until the coughing or gasping has stopped or subsided since the coughing or gasping may affect the accuracy of the other parameters determined from the accelerometer signal.

As noted above, there are a number of ways in which a COPD severity score can be formed from the measurements determined in step 101.

A scoring system to assess respiratory function and COPD severity can be developed by classifying the measured parameters (i.e. activity level, breathing rate, respiratory effort and measure of the severity or intensity of coughing) based on a simplified version of the BODE score. Preferably, baselining and calibration of the COPD severity score according to the invention against the existing BODE score can result in both scores providing a similar clinical value (although the COPD severity score according to the invention is more readily obtainable).

The table in Figure 4 illustrates how parameters can be weighed and combined to assess COPD severity according to an exemplary embodiment of the invention. In this embodiment, for each parameter measurement, the deviation with respect to a desired 'normal' range is scored (e.g. given a value of 0, 1, 2 or 3), which is also referred to as a 'severity range', and the scores are added to arrive at a COPD severity score. It will be appreciated that the range of parameter values within each level of severity (e.g. 600-899 activity counts for a score of 1) illustrated in Figure 4 is exemplary and other ranges can be used.

An exemplary system for the COPD severity score could be: very mild COPD (0-3 points), mild COPD (4-6 points), moderate COPD (7-9 points), and severe COPD (>9 points).

It will be appreciated that there are alternative ways of calculating the COPD severity score to the use of the table shown in Figure 4. For example, analysis of the parameters to determine an indication of the COPD severity can use a classification algorithm (such as a Bayesian classification algorithm or a neural network) to adaptively weight the parameter values in order to determine the COPD severity.

Two ways of determining the 'normal' and other severity ranges for each parameter are set out below.

In a first technique, an initial clinical diagnosis by a general practitioner (GP) of COPD based on the clinically accepted standard BODE score (obtained using traditional approaches with multiple sensors) is compared to a BODE-equivalent score obtained from measurements of the four parameters (along with measurements of FEV and BMI). The derivation of a BODE-equivalent score from the measurements of the four parameters used herein is described in more detail below. The standard (full) BODE score has established ranges for each parameter (FEV, BMI, 6MWD and Dyspnea) as shown in Figure 1. By comparing a BODE-equivalent score obtained from the four parameters, the ranges in the full BODE score will also be intrinsically incorporated into the BODE equivalent COPD severity score. Adjustment or calibration of the BODE score obtained from the four parameters can be achieved, for example, by finding the ratio of the BODE score obtained in the standard manner to the BODE score obtained from the four parameters measured according to the invention, and using the ratio as a multiplication factor for the equivalent BODE score obtained using the invention. This ratio can be adjusted each time that a new BODE score is obtained using the standard approach during a clinical visit. Those skilled in the art will be aware of other ways of calibrating the equivalent-BODE score obtained using the invention to the standard BODE score. In one alternative, the calibration can be based on individual parameter weighting factors that are derived, for example, by calibrating the activity level to the 6-minute walking distance, and calibrating the breathing rate and respiratory effort to the MMRC Dyspnea scale (FEV and BMI are the same for both the standard BODE score and the equivalent BODE score obtained using the invention). In this way the COPD severity scoring system according to the invention is calibrated and highly correlated with the standard BODE score.

Moreover, since COPD is a progressive, degenerative respiratory disease with no cure, the respiratory function of the subject will, without exception, decline over time. Thus each parameter measurement obtained during use of the apparatus 2 can be normalized using the initial or baseline value obtained by the apparatus 2 at the initial clinical diagnosis (and compared to the full BODE score obtained in the conventional manner) or at the first use of the apparatus 2. 'Baselining' also ensures that a subject-specific COPD severity score is obtained since it allows the current COPD severity of a subject to be compared to their initial baseline severity score, which means that at any given time an assessment can be made as to how severe or mild the COPD symptoms of the subject are (i.e. an assessment of the disease progression). If this was not done, then a subject can be compared to a statistical average of the COPD population, although this may not allow small fluctuations or degradations in COPD severity to be detected.

In a second technique, statistical analysis (e.g. using a bias corrected and accelerated bootstrap method, Lilliefors testing, Shapiro-Wilk W testing, principal component analysis, etc.) of a database of data for a set of subjects with COPD and/or of an elderly population group, including healthy individuals and subjects with COPD can be used to identify suitable ranges of values. This analysis can also compare factors such as age and ambulatory status (e.g. does the subject use a walking aid) and the results of this comparison can be used to further refine these ranges to ensure appropriate specificity.

In some embodiments, regardless of how the normal and increasing severity ranges are determined, the ranges for some or all of the parameters can be set or adjusted based on the ambulatory state or capability of the subject, which means that different subjects can have different normal values/ranges. In particular a subject that uses equipment to assist them to walk, such as a frame or walking stick, will have a different ambulatory capability than subjects that are able to walk unassisted. In these embodiments it is therefore important to take the ambulatory state of the subject into account when assessing the COPD severity in order to arrive at an appropriate value for the COPD severity score. Since the ambulatory state of the subject (for example in terms of whether walking aids are required) is not something that is typically expected to vary day-to-day, the ambulatory state of the subject can be input to the apparatus 2 during a set-up or calibration phase. For a less-capable subject the normal value ranges for activity count may be at least 50% less than for a more-capable subject, and the scoring ranges for the activity count parameter can be adjusted accordingly. Other parameters, such as the measure of respiratory effort, are not affected as significantly, but some smaller adjustment to the scoring ranges can be made if required.

Based on the COPD severity score determined according to the invention, an appropriate clinical intervention can be made. This may involve a clinician adjusting the dosing of the bronchodilators or adjusting the timing of the dose or the timely administration of corticosteroids to avert a predicted exacerbation event. It may also or alternatively involve providing the subject with an indication that an exacerbation event may occur and advising them to take a dose of bronchodilators.

In further or alternative embodiments, in addition to determining the COPD severity score according to the invention, the apparatus 2 can also be used to derive a 'full' BODE score (e.g. as shown in Figure 1). In this case, either the apparatus 2 can be provided with measurements of the BMI and the FEV for the subject (for example by the subject or a clinician manually inputting them into the apparatus 2) and the apparatus 2 can calculate the full BODE score, or the parameters measured by the apparatus 2 can be output to another device which can calculate the BODE score. It will be appreciated that the BMI and FEV are measurements that are routinely taken by clinicians during periodic patient check-up visits. The 6-minute Walking Distance and MMRC Dyspnea Scale required for the full BODE score can be derived from the activity level, position tracking (if the apparatus 2 includes a suitable sensor for determining the location or position of the subject) and breathing rate measurements obtained through the day, e.g. during dressing and undressing, when walking around the house or when walking to and from the supermarket. In some cases initial starting input values may be used by the device to generate an initial BODE score prior to optimization or personalization for the subject. Over time as measurements are acquired by the apparatus 2 it will generate a more optimal or personalized score. Other subject-specific starting settings might include the timing of generating the unobtrusive COPD severity score. For example, if a subject typically goes for a walk at a specific time of day, the apparatus 2 can schedule a COPD severity score measurement at that time.

As noted above, in further embodiments, the apparatus 2 can include additional sensors to the accelerometer 4 for measuring other parameters that can improve the assessment of the COPD severity or of the general health of the subject. One such sensor is a temperature sensor, e.g. a zero heat flux temperature sensor, which can be used to detect changes in the body temperature of the subject that can occur when a respiratory infection occurs. The measurements from the temperature sensor can be used in the clinical management of COPD patients (for example to determine whether to adjust a medication level or determine that a new medication may be required, e.g. in the case of a respiratory infection).

In further or alternative embodiments, the apparatus 2 can improve the assessment of the activity level of the subject beyond a simple activity count. In some embodiments, the control unit 6 can implement an activity classifier algorithm to attempt to classify the activity that the subject is engaging in (e.g. sitting down, lying down, walking, exercising, etc.). Suitable classifier algorithms will be known to those skilled in the art. In some embodiments, the apparatus 2 can include a satellite position system receiver to track the location of the subject (and thus also provide an indication of the speed of movement of the subject). This location and speed information can be used to improve the estimate of the activity being performed by the subject, since some activities, e.g. bicycle riding, will provide a low activity count according to the technique described above since only the subject's legs are moving. This will greatly improve the reliability of the device.

Although in preferred embodiments the parameters are measured continuously or frequently throughout the day, it will be appreciated that it is possible in some circumstances that values for one or more parameters cannot be calculated by the apparatus 2. This means that the COPD severity score cannot be calculated at that time. Instead, values for the missing parameters can be input to the apparatus 2 at a later stage to enable the calculation of the COPD severity score to be completed.

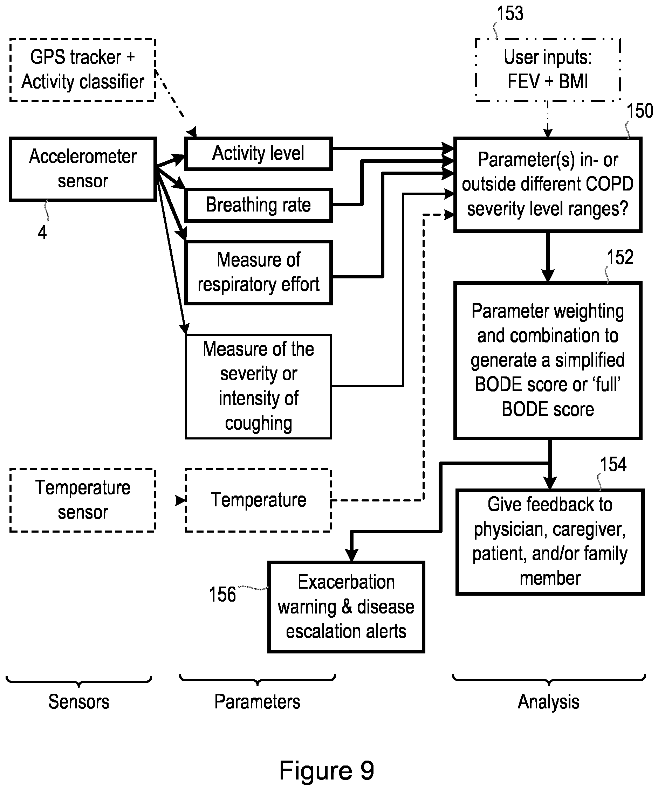

Figure 9 provides an overview of various ones of the embodiments described above. Figure 9 shows the sensors that can be provided in the apparatus 2, the parameters that can be derived from the sensor measurements, and the processing steps or analysis that can be performed on the sensor measurements. The accelerometer 4 and parameters derived from the acceleration measurements (activity level, breathing rate, measure of the respiratory effort and measure of the severity or intensity of coughing) are shown with a solid outline to indicate that they are used in all embodiments, and the other sensors (GPS tracker, activity classifier and temperature sensor) and parameter (temperature) are shown with a dashed outline to indicate that they are optional features. Thus, as described above, the parameters derived from the acceleration measurements are compared to respective severity ranges to determine a COPD severity score (step 150), and the parameters or scores can be weighted and/or combined in step 152 to generate a simplified BODE score or a full BODE score (if measurements of FEV and BMI are provided - step 153). Feedback on the score can then be provided to the clinician or subject or another person (step 154), and, if necessary, a warning of an imminent exacerbation can be given.

As noted above, the invention provides an apparatus and computer program product which determines measurements of several physiological characteristics of a subject that are clinical indicators of COPD severity (breathing rate, activity level, measure of respiratory effort and measure of the severity or intensity of coughing and that combines them into a single COPD severity score. The nature of the physiological characteristics used to form the score means that it is possible to continuously, automatically and reliably monitor or indicate COPD severity and respiratory function for the subject. The apparatus and computer program product according to the invention improves the clinical management of subjects suffering from COPD. In addition, the apparatus and computer program product may also be useful for the monitoring of other respiratory diseases including pneumonia, tuberculosis, emphysema, and chronic bronchitis, among others.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer program product for assessing the severity of chronic obstructive pulmonary disease, COPD, in a subject, the computer program product comprising a computer readable medium having computer readable code embodied therein, the computer readable code being configured such that, on execution on the control unit of an apparatus or system of any of claims 10-14, the apparatus or system caused to perform the steps of:
determining (101) by means of an accelerometer (4) measurements of a breathing rate of the subject, an activity level of the subject, a measure of the respiratory effort of the subject and a measure of the severity or intensity of coughing by the subject; and
combining (103) the measurements of the breathing rate, the activity level, the measure of the respiratory effort and the measure of the severity or intensity of coughing to determine a score representing the severity of COPD in the subject.

2. The computer program product as claimed in claim 1, wherein the breathing rate of the subject, the activity level of the subject, the measure of the respiratory effort of the subject and the measure of the severity or intensity of coughing by the subject are determined from measurements of the accelerometer (4) only.

3. The computer program product as claimed in claim 1, wherein the breathing rate of the subject, the activity level of the subject, the measure of the respiratory effort of the subject and the measure of the severity or intensity of coughing by the subject are determined from measurements of the accelerometer (4) and from measurements of at least one additional sensor.

4. The computer program product, wherein the step of combining (103) the measurements to determine a score representing the severity of COPD in the subject comprises:
comparing each of the measurements of the breathing rate, the activity level, the measure of the respiratory effort and the measure of the severity or intensity of coughing to a respective severity range to determine a score for each of the measurements; and
adding the determined scores to determine the score representing the severity of COPD in the subject.

5. The computer program product as claimed in any of claims 1-4, the method further comprising the step of calibrating the score representing the severity of COPD in the subject against a Body mass index, Obstruction, Dyspnea and Exercise, BODE, score.

6. The computer program product as claimed in any of claims 1-5, the method further comprising the step of:
comparing the measure of the intensity or severity of coughing to a threshold value to determine if there is a high risk of an exacerbation event or if an exacerbation event is ongoing.

7. The computer program product as claimed in any of claims 1-6, the method further comprising the steps of:
detecting when the subject coughs or gasps; and
temporarily interrupting the step of determining measurements or the use of the measurements in the step of combining when the subject coughs or gasps.

8. An apparatus (2) for assessing the severity of chronic obstructive pulmonary disease, COPD, in a subject, the apparatus (2) comprising:
a control unit (6) configured to determine by means of an accelerometer (4) measurements of a breathing rate of the subject, an activity level of the subject, a measure of the respiratory effort and a measure of the intensity or severity of coughing, and to combine the measurements of the breathing rate, the activity level, the measure of respiratory effort and the measure of the intensity or severity of coughing to determine a score representing the severity of COPD in the subject.

9. An apparatus (2) as claimed in claim 8, wherein the control unit (6) is configured to determine the measurements of the breathing rate of the subject, the activity level of the subject, the measure of the respiratory effort of the subject and the measure of the severity or intensity of coughing by the subject from measurements from the accelerometer (4) only.

10. An apparatus (2) as claimed in claim 8 or 9, the apparatus (2) further comprising the accelerometer (4).

11. An apparatus (2) as claimed in claim 8, wherein the apparatus (2) comprises the accelerometer (4) and at least one additional sensor, wherein the control unit (6) is configured to determine the measurements of the breathing rate of the subject, the activity level of the subject, the measure of the respiratory effort of the subject and the measure of the severity or intensity of coughing by the subject from measurements from the accelerometer (4) and the at least one additional sensor.

12. An apparatus (2) as claimed in claim 11, wherein the at least one additional sensor is a sensor for measuring the motion and/or position of the subject.

13. A system for assessing the severity of chronic obstructive pulmonary disease, COPD, in a subject, the system comprising:
the accelerometer (4) for measuring characteristics of the subject; and
an apparatus (2) as claimed in claims 8 or 9.

14. A system as claimed in claim 13, further comprising at least one additional sensor for measuring the motion and/or position of the subject.

## Patentansprüche

1. Ein Computerprogrammprodukt zum Bewerten des Schweregrads der chronisch obstruktiven Lungenerkrankung (COPD) eines Patienten, wobei das Computerprogrammprodukt ein von einem Computer lesbares Medium mit von einem Computer lesbaren Code umfasst, wobei der von einem Computer lesbare Code beim Ausführen auf der Steuerung eines Geräts oder Systems gemäß einem der Ansprüche 10 bis 14 das Gerät oder das System dazu veranlasst, die folgenden Schritte durchzuführen:
Ermitteln (101) von Messungen der Atemfrequenz des Patienten, des Aktivitätsniveaus des Patienten, des Grads der Atmungsanstrengung des Patienten und des Schweregrads oder der Intensität des Hustens des Patienten mithilfe eines Beschleunigungsmessers (4); und
Kombinieren (103) der Messungen der Atemfrequenz, des Aktivitätsniveaus, des Grads der Atmungsanstrengung und des Schweregrads oder der Intensität des Hustens, um einen Wert zu ermitteln, der den Schweregrad der COPD des Patienten angibt.

2. Das Computerprogrammprodukt gemäß Anspruch 1, wobei die Atemfrequenz des Patienten, das Aktivitätsniveau des Patienten, der Grad der Atmungsanstrengung des Patienten und der Schweregrad oder die Intensität des Hustens des Patienten ausschließlich anhand der Messungen des Beschleunigungsmessers (4) ermittelt werden.

3. Das Computerprogrammprodukt gemäß Anspruch 1, wobei die Atemfrequenz des Patienten, das Aktivitätsniveau des Patienten, der Grad der Atmungsanstrengung des Patienten und der Schweregrad oder die Intensität des Hustens des Patienten anhand der Messungen des Beschleunigungsmessers (4) sowie anhand der Messungen mindestens eines weiteren Sensors ermittelt werden.

4. Das Computerprogrammprodukt, wobei das Kombinieren (103) der Messungen zum Ermitteln eines Wertes, der den Schweregrad der COPD des Patienten angibt, folgende Schritte umfasst:
Vergleichen der einzelnen Messungen der Atemfrequenz, des Aktivitätsniveaus, des Grads der Atmungsanstrengung und des Schweregrads oder der Intensität des Hustens mit einem jeweiligen Schwerebereich, um einen Wert für die einzelnen Messungen zu ermitteln; und
Addieren der ermittelten Werte, um den Wert zu ermitteln, der den Schweregrad der COPD des Patienten angibt.

5. Das Computerprogrammprodukt gemäß einem der Ansprüche 1 bis 4, wobei die Methode zudem das Kalibrieren des den Schweregrad der COPD beim Patienten angebenden Werts anhand des BODE-Index für Body-Mass-Index, Einsekundenkapazität, Luftnot und Gehtest umfasst.

6. Das Computerprogrammprodukt gemäß einem der Ansprüche 1 bis 5, wobei die Methode zudem folgenden Schritt umfasst:
Vergleichen des Maßes für die Intensität oder den Schweregrad des Hustens mit einem Schwellenwert, um zu ermitteln, ob ein hohes Risiko für eine Verschlimmerung besteht oder ob diese im Gang ist.

7. Das Computerprogrammprodukt gemäß einem der Ansprüche 1 bis 6, wobei die Methode zudem folgende Schritte umfasst:
Erkennen, wenn der Patient hustet oder keucht; und
vorübergehendes Unterbrechen des Ermittelns von Messungen oder des Verwendens der Messungen für das Kombinieren, wenn der Patient hustet oder keucht.

8. Ein Gerät (2) zum Bewerten des Schweregrads der chronisch obstruktiven Lungenerkrankung (COPD) eines Patienten, wobei das Gerät (2) Folgendes umfasst:
eine Steuerung (6), die folgende Schritte durchführt: Ermitteln von Messungen der Atemfrequenz des Patienten, des Aktivitätsniveaus des Patienten, des Grads der Atmungsanstrengung und des Schweregrads oder der Intensität des Hustens mithilfe eines Beschleunigungsmessers (4), und Kombinieren der Messungen der Atemfrequenz, des Aktivitätsniveaus, des Grads der Atmungsanstrengung und des Schweregrads oder der Intensität des Hustens, um einen Wert zu ermitteln, der den Schweregrad der COPD des Patienten angibt.

9. Ein Gerät (2) gemäß Anspruch 8, wobei die Steuerung (6) die Atemfrequenz des Patienten, das Aktivitätsniveau des Patienten, den Grad der Atmungsanstrengung des Patienten und den Schweregrad oder die Intensität des Hustens des Patienten ausschließlich anhand der Messungen des Beschleunigungsmessers (4) ermittelt.

10. Ein Gerät (2) gemäß Anspruch 8 oder 9, wobei das Gerät (2) zudem einen den Beschleunigungsmesser (4) umfasst.

11. Ein Gerät (2) gemäß Anspruch 8, wobei das Gerät (2) den Beschleunigungsmesser (4) und mindestens einen zusätzlichen Sensor umfasst, wobei die Steuerung (6) die Atemfrequenz des Patienten, das Aktivitätsniveau des Patienten, den Grad der Atmungsanstrengung des Patienten und den Schweregrad oder die Intensität des Hustens des Patienten anhand der Messungen des Beschleunigungsmessers (4) und des mindestens einen zusätzlichen Sensors ermittelt.

12. Ein Gerät (2) gemäß Anspruch 11, wobei es sich beim mindestens einen zusätzlichen Sensor um einen Sensor zum Messen der Bewegungen und/oder der Position des Patienten handelt.

13. Ein System zum Bewerten des Schweregrads der chronisch obstruktiven Lungenerkrankung (COPD) eines Patienten, wobei das System Folgendes umfasst:
den Beschleunigungsmesser (4) zum Messen der Eigenschaften des Patienten; und
ein Gerät (2) gemäß Anspruch 8 oder 9.

14. Ein System gemäß Anspruch 13, das zudem mindestens einen zusätzlichen Sensor zum Messen der Bewegungen und/oder der Position des Patienten umfasst.

## Revendications

1. Programme informatique servant à évaluer la gravité de la maladie pulmonaire obstructive chronique, BPCO, chez un sujet, le programme informatique comprenant un support lisible par ordinateur ayant un code lisible par ordinateur intégré dans celui-ci, le code lisible par ordinateur étant configuré de sorte que lorsqu'il est exécuté sur l'unité de commande d'un appareil ou d'un système de l'une quelconque des revendications 10 à 14, l'appareil ou le système est amené à réaliser les étapes de :
détermination (101) au moyen d'un accéléromètre (4) des mesures du rythme respiratoire du sujet, d'un niveau d'activité du sujet, d'une mesure de l'effort respiratoire du sujet et d'une mesure de la gravité ou de l'intensité de la toux du sujet ; et
combinaison (103) des mesures du rythme respiratoire, du niveau d'activité, de la mesure de l'effort respiratoire et de la mesure de la gravité ou de l'intensité de la toux pour déterminer un score représentant la gravité de la BPCO chez le sujet.

2. Programme informatique selon la revendication 1, dans lequel le rythme respiratoire du sujet, le niveau d'activité du sujet, la mesure de l'effort respiratoire du sujet et la mesure de la gravité ou de l'intensité de la toux du sujet sont déterminés à partir des mesures de l'accéléromètre (4) uniquement.

3. Programme informatique selon la revendication 1, dans lequel le rythme respiratoire du sujet, le niveau d'activité du sujet, la mesure de l'effort respiratoire du sujet et la mesure de la gravité ou de l'intensité de la toux du sujet sont déterminés à partir des mesures de 1' accéléromètre (4) et des mesures d'au moins un capteur supplémentaire.

4. Programme informatique, dans lequel l'étape de combinaison (103) des mesures pour déterminer un score représentant la gravité de la BPCO chez le sujet comprend :
la comparaison de chacune des mesures du rythme respiratoire, du niveau d'activité, de la mesure de l'effort respiratoire et de la mesure de la gravité ou de l'intensité de la toux avec une plage de gravité afin de déterminer un score pour chacune des mesures ; et
l'ajout des scores déterminés pour déterminer le score représentant la gravité de la BPCO chez le sujet.

5. Programme informatique selon l'une quelconque des revendications 1 à 4, le procédé comprenant en outre l'étape d'étalonnage du score représentant la gravité de la BPCO chez le sujet par rapport à l'indice de masse corporelle, au score d'obstruction, de dyspnée et de tolérance à l'exercice, évalués dans le cadre de l'indice BODE.

6. Programme informatique selon l'une quelconque des revendications 1 à 5, le procédé comprenant en outre l'étape de :
comparaison de la mesure de gravité ou d'intensité de la toux par rapport à une valeur seuil pour déterminer s'il existe un risque élevé d'événement d'exacerbation ou si un événement d'exacerbation est en cours.

7. Programme informatique selon l'une quelconque des revendications 1 à 6, le procédé comprenant en outre les étapes consistant à :
détecter lorsque le sujet tousse ou halète ; et
interrompre temporairement l'étape de détermination des mesures ou de l'utilisation des mesures dans l'étape de combinaison lorsque le sujet tousse ou halète.

8. Appareil (2) permettant d'évaluer la gravité de la maladie pulmonaire obstructive chronique, BPCO, chez un sujet, l'appareil (2) comprenant :
une unité de commande (6) configurée pour déterminer, au moyen d'un accéléromètre (4) les mesures d'un rythme respiratoire du sujet, un niveau d'activité du sujet, une mesure de l'effort respiratoire et une mesure de l'intensité ou de la gravité de la toux, et pour combiner les mesures du rythme respiratoire, du niveau d'activité, la mesure de l'effort respiratoire et la mesure de l'intensité ou de la gravité de la toux afin de déterminer un score représentant la gravité de la BPCO chez le sujet.

9. Appareil (2) selon la revendication 8, dans lequel l'unité de commande (6) est configurée pour déterminer les mesures du rythme respiratoire du sujet, le niveau d'activité du sujet, la mesure de l'effort respiratoire du sujet et la mesure de la gravité ou de l'intensité de la toux du sujet à partir des mesures de l'accéléromètre (4) seulement.

10. Appareil (2) selon la revendication 8 ou 9, l'appareil (2) comprenant en outre l'accéléromètre (4) .

11. Appareil (2) selon la revendication 8, dans lequel l'appareil (2) comprend l'accéléromètre (4) et au moins un capteur supplémentaire, dans lequel l'unité de commande (6) est configurée pour déterminer les mesures du taux respiratoire du sujet, le niveau d'activité du sujet, la mesure de l'effort respiratoire du sujet et la mesure de la gravité ou de l'intensité de la toux du sujet à partir des mesures de l'accéléromètre (4) et d'au moins un capteur supplémentaire.

12. Appareil (2) selon la revendication 11, dans lequel au moins un capteur supplémentaire est un capteur pour mesurer le mouvement et/ou la position du sujet.

13. Système pour évaluer la gravité de la maladie pulmonaire obstructive chronique, BPCO, chez un sujet, le système comprenant :
l'accéléromètre (4) pour mesurer les caractéristiques du sujet ; et
un appareil (2) selon les revendications 8 ou 9.

14. Système selon la revendication 13, comprenant en outre au moins un capteur supplémentaire pour mesurer le mouvement et/ou la position du sujet.
